# EUROPÄISCHE PATENTSCHRIFT

(11) **EP 4 257 063 B1**
(45) Veröffentlichungstag und Bekanntmachung des Hinweises auf die Patenterteilung: **11.11.2026**
(21) Anmeldenummer: 23167042.3
(22) Anmeldetag: 06.04.2023
(51) Int. Cl.: A61B 17/16, A61F 2/36, A61F 2/46, A61B 17/00, A61F 2/30, A61B 90/00

(54) **MEDIZINISCHES INSTRUMENTARIUM UND GELENKIMPLANTATIONSSYSTEM**
MEDICAL INSTRUMENTATION AND ARTICULATION IMPLANT SYSTEM
INSTRUMENT MÉDICAL ET SYSTÈME D'IMPLANTATION ARTICULAIRE

(30) Priorität: 08.04.2022 DE 102022108553
(43) Veröffentlichungstag der Anmeldung: 11.10.2023
(73) Patentinhaber: Aesculap AG, 78532 Tuttlingen (DE)
(72) Erfinder: Saueressig, Thomas, 78532 Tuttlingen (DE)
(74) Vertreter: Hoeger, Stellrecht & Partner Patentanwälte mbB

(56) Entgegenhaltungen:
- EP-A2- 2 777 550
- EP-B1- 1 905 394
- US-A1- 2012 259 338

## Beschreibung

Die vorliegende Erfindung betrifft ein medizinisches Instrumentarium, umfassend ein Spanwerkzeug und eine Konusschutzeinrichtung, wobei die Konusschutzeinrichtung eine eine Längsachse definierende Konusschutzhülse umfasst und ausgebildet ist zum lösbaren Verbinden mit einer einen Verbindungskonus aufweisenden ersten Implantatkomponente einer Gelenkendoprothese derart, dass die Konusschutzhülse in einer Verbindungsstellung, in welcher die Konusschutzeinrichtung und die erste Implantatkomponente miteinander kraft- und/oder formschlüssig in Eingriff stehen, den Verbindungskonus mindestens teilweise, insbesondere vollständig, umgibt und wobei das Spanwerkzeug die Konusschutzeinrichtung und den Verbindungskonus in einer Bearbeitungsstellung umgibt.

Ferner betrifft die vorliegende Erfindung ein Gelenkimplantatsystem umfassend eine in eine Knochenkavität implantierbare erste Implantatkomponente einer modularen Gelenkendoprothese, wobei die erste Implantatkomponente einen sich in proximaler Richtung verjüngenden Verbindungskonus umfasst zum klemmenden Verbinden mit einer zweiten Implantatkomponente der modularen Gelenkendoprothese.

Ein medizinisches Instrumentarium und ein Gelenkimplantationssystem der eingangs beschriebenen Art sind beispielsweise aus der EP 1 905 394 B1 bekannt. Das bekannte medizinische Instrumentarium dient insbesondere dem Zweck, einen proximalen Bereich eines Hohlraums, also einer Kavität in einem Knochen, zum Implantieren der ersten Implantatkomponente durch Räumen für die Implantation vorzubereiten.

Ein Problem bei dem bekannten Instrumentarium ist insbesondere, dass sich die Konusschutzhülse nur unmittelbar über den Verbindungskonus der ersten Implantatkomponente erstreckt. Dies erschwert eine Handhabung des Spanwerkzeugs durch einen Operateur, und zwar insbesondere dann, wenn der chirurgische Eingriff minimalinvasiv vorgenommen werden soll.

Aus der EP 2 777 550 A2 ist ein orthopädisches chirurgische Instrument bekannt. Ein distaler Reamer zur Verwendung bei einem orthopädischen chirurgischen Eingriff zur Implantation einer Hüftgelenk-Revisionsprothese ist in der US 2012/0259338 A1 beschrieben.

Es ist daher eine Aufgabe der vorliegenden Erfindung, ein medizinisches Instrumentarium und ein Gelenkimplantationssystem der eingangs beschriebenen Art so zu verbessern, dass sie einfacher handhabbar sind.

Diese Aufgabe wird bei einem medizinischen Instrumentarium der eingangs beschriebenen Art erfindungsgemäß dadurch gelöst, dass das Spanwerkzeug und die Konusschutzeinrichtung zusammenwirkend ausgebildet sind zum Führen einer Bewegung des Spanwerkzeugs parallel zur und um die Längsachse relativ zur Konusschutzeinrichtung.

Eine solche Ausgestaltung ermöglicht es insbesondere, das Spanwerkzeug beim Räumen des proximalen Bereichs der Kavität optimal durch die Konusschutzeinrichtung zu führen. Eine solche Führungsfunktion kann insbesondere auf einer gesamten Länge der Konusschutzhülse realisiert werden und insbesondere auch auf einer gesamten oder im Wesentlichen gesamten Länge der Konusschutzeinrichtung. Mit anderen Worten sind also auch das Spanwerkzeug und die von der Konusschutzeinrichtung umfasste Konusschutzhülse zusammenwirkend ausgebildet zum Führen einer Bewegung des Spanwerkzeugs parallel zur und um die Längsachse relativ zur von der Konusschutzeinrichtung umfassten Konusschutzhülse. Zudem kann das Führen des Spanwerkzeugs durch die Konusschutzeinrichtung insbesondere dadurch verbessert werden, dass die Konusschutzeinrichtung mit einem proximalen Ende über ein proximales Ende des Spanwerkzeugs vorsteht. Dies ermöglicht es weiterhin, das Spanwerkzeug und die Konusschutzeinrichtung optional so miteinander zu verbinden, dass sie relativ zueinander parallel zur und um die Längsachse verschieb- und verdrehbar sind. So kann ein Operateur in definierter Weise den proximalen Bereich der Kavität bearbeiten, auch ohne direkte Sicht auf den Operationssitus zu haben. Die Konusschutzeinrichtung führt das Spanwerkzeug in definierter Weise, beispielsweise durch miteinander zusammenwirkende Flächen oder Führungselemente, die einerseits am Spanwerkzeug und andererseits an der Konusschutzeinrichtung, insbesondere an der Konusschutzhülse, angeordnet oder ausgebildet sind. Es ist also insbesondere auch eine Führung des Spanwerkzeugs durch die Konusschutzhülse möglich, und zwar insbesondere auf der gesamten Länge derselben, also von einem proximalen Ende bis zu einem distalen Ende der Konusschutzhülse. Auf diese Weise kann eine Handhabung des medizinischen Instrumentariums für einen Operateur signifikant verbessert werden.

Um eine Beschädigung der ersten Implantatkomponente durch das Spanwerkzeug zu vermeiden, ist es günstig, wenn die Konusschutzeinrichtung einen Tiefenanschlag umfasst zum Begrenzen einer Bewegung des Spanwerkzeugs parallel zur Längsachse in distaler Richtung. Insbesondere wenn am Spanwerkzeug distalseitig Schneidkanten angeordnet oder ausgebildet sind, um eine Knochenkavität, in welche die erste Implantatkomponente der Gelenkendoprothese eingesetzt werden soll, zu bearbeiten, kann so verhindert werden, dass das Spanwerkzeug mit der ersten Implantatkomponente in Kontakt treten und diese beschädigen kann.

Vorteilhaft ist es, wenn der Tiefenanschlag eine in proximaler Richtung weisende erste Anschlagfläche umfasst. Insbesondere kann die erste Anschlagfläche in Form einer ersten Ringfläche ausgebildet sein. An der ersten Anschlagfläche kann insbesondere ein am Spanwerkzeug angeordneter oder ausgebildeter Versatz anschlagen, wenn dieses relativ zur Konusschutzeinrichtung seine maximal weit in distaler Richtung vorbewegte Stellung einnimmt.

Vorzugsweise sind das Spanwerkzeug und die Konusschutzeinrichtung unverlierbar aneinander gesichert. Diese Ausgestaltung hat insbesondere den Vorteil, dass ein Operateur das Spanwerkzeug nicht mit der Konusschutzeinrichtung in Eingriff bringen muss. Vielmehr kann er die Konusschutzeinrichtung mit dem an dieser unverlierbar gesicherten Spanwerkzeug mit der ersten Implantatkomponente koppeln und dann durch einfache Bewegung des Spanwerkzeugs und der Konusschutzeinrichtung relativ zueinander den proximalen Bereich der Kavität in gewünschter Weise präparieren. Anders als bei dem aus der EP 1 905 394 B1 bekannten Instrumentarium ist daher die Kopplung des Spanwerkzeugs mit der Konusschutzhülse nicht erforderlich. Insbesondere bei minimalinvasiven Eingriffen kann so eine Handhabung des medizinischen Instrumentariums verbessert werden. Durch die unverlierbare Sicherung von Spanwerkzeug und Konusschutzeinrichtung aneinander kann ein Anwender nicht vergessen, mit der Konusschutzeinrichtung den Verbindungskonus zu schützen und ohne diesen zu bohren oder zu spanen. Somit wird durch die vorgeschlagene Weiterbildung auch die Gefahr verringert, dass der Verbindungskonus der ersten Implantatkomponente beschädigt werden kann, was wiederum zu einem Bruch derselben führen kann. Dies kann mit einem wie vorgeschlagen weitergebildeten Instrumentarium nicht passieren.

Günstig ist es, wenn die Konusschutzeinrichtung einen proximalen Anschlag zum Begrenzen einer Bewegung des Spanwerkzeugs parallel zur Längsachse in proximaler Richtung umfasst. Durch das Vorsehen des Tiefenanschlags in Verbindung mit dem proximalen Anschlag kann das Spanwerkzeug insbesondere unverlierbar an der Konusschutzeinrichtung gesichert werden. Das Spanwerkzeug kann dann zwischen einer proximalen und einer distalen Extremstellung bewegt werden relativ zur Konusschutzeinrichtung. Ein Hub des Spanwerkzeugs relativ zur Konusschutzeinrichtung wird dann bestimmt durch einen Abstand des Tiefenanschlags und des proximalen Anschlags voneinander abzüglich eines Abstands der mit diesen beiden Anschlägen zusammenwirkenden Elementen am Spanwerkzeug voneinander.

Auf einfache Weise lässt sich der proximale Anschlag ausbilden, wenn er eine in distaler Richtung weisende zweite Anschlagfläche umfasst. Insbesondere kann diese auch in Form einer zweiten Ringfläche ausgebildet sein. Beispielsweise kann der proximale Anschlag durch ein Handhabungselement gebildet werden, welches mit der Konusschutzeinrichtung verbunden wird. Eine unverlierbare Sicherung kann dann beispielsweise dadurch erreicht werden, dass das Handhabungselement dauerhaft unlösbar mit der Konusschutzeinrichtung verbunden wird. Unlösbar in diesem Sinne bedeutet, dass die Konusschutzeinrichtung und das Spanwerkzeug nur voneinander getrennt werden können, indem eines dieser beiden Teile ganz oder teilweise zerstört wird, sodass es für einen bestimmungsgemäßen Gebrauch nicht mehr geeignet ist.

Günstigerweise ist die Konusschutzhülse derart bemessen, dass sie den Verbindungskonus in der Verbindungsstellung nicht berührt. Dies kann insbesondere dadurch erreicht werden, dass zwischen einer in Richtung auf den Verbindungskonus weisenden Konusschutzhülseninnenfläche und dem Verbindungskonus in der Verbindungstellung allseitig ein definierter Abstand vorgegeben wird. Auf diese Weise kann im Vergleich zu dem aus der EP 1 905 394 B1 bekannten Instrumentarium eine Beschädigung des Verbindungskonus der ersten Implantatkomponente vermieden werden, da bei dem vorgeschlagenen medizinischen Instrumentarium keine Berührung zwischen der Konusschutzhülse der Konusschutzeinrichtung und dem Verbindungskonus möglich ist.

Gemäß einer weiteren bevorzugten Ausführungsform der Erfindung kann vorgesehen sein, die Konusschutzeinrichtung ein Konusschutzeinrichtungkopplungselement umfasst zum kraft- und/oder formschlüssigen Koppeln mit einem korrespondierenden Implantatkomponentenkopplungselement der ersten Implantatkomponente der Gelenkendoprothese in der Verbindungsstellung. Die in der Verbindungsstellung zusammenwirkenden Konusschutzeinrichtungkopplungs- und Implantatkomponentenkopplungselemente ermöglichen eine definierte und sichere Verbindung der Konusschutzeinrichtung mit der ersten Implantatkomponente, um den Verbindungskonus in definierter Weise zu schützen. Insbesondere kann die Ausgestaltung des Konusschutzeinrichtungkopplungselements beziehungsweise des Implantatkomponentenkopplungselements eine definierte Relativposition zwischen der Konusschutzeinrichtung und der ersten Implantatkomponente in der Verbindungsstellung definierter Weise vorgeben.

Auf einfache Weise lassen sich die Konusschutzeinrichtung und die erste Implantatkomponente miteinander koppeln, wenn das Konusschutzeinrichtungkopplungselement in Form eines Innen- oder Außengewindeabschnitts ausgebildet ist. So kann es beispielsweise mit einem korrespondierenden Außen- oder Innengewindeabschnitt an der ersten Implantatkomponente verschraubt werden.

Günstig ist es, wenn die die Konusschutzeinrichtung eine erste Führungsfläche zum Führen einer Bewegung des Spanwerkzeugs parallel zur und um die Längsachse relativ zur Konusschutzeinrichtung umfasst und wenn die erste Führungsfläche durch eine Außenfläche der Konusschutzeinrichtung gebildet ist. Mithin lässt sich also das Spanwerkzeug bei dieser Ausgestaltung entlang der Außenfläche der Konusschutzeinrichtung führen. Hierfür kann das Spanwerkzeug mit einer zur Außenfläche korrespondierenden Fläche an der Konusschutzeinrichtung entlanggleiten.

Vorteilhaft ist es, wenn das Spanwerkzeug eine zweite Führungsfläche umfasst, wenn die zweite Führungsfläche korrespondierend zur ersten Führungsfläche ausgebildet ist und wenn die zweite Führungsfläche durch eine auf die Längsachse hin weisende Innenfläche des Spanwerkzeugs ausgebildet ist. Diese Ausgestaltung des Spanwerkzeugs ermöglicht insbesondere eine optimale und sichere Führung des Spanwerkzeugs bei einer Bewegung relativ zur Konusschutzeinrichtung.

Eine einfache und sichere Führung kann insbesondere dadurch erreicht werden, dass die erste Führungsfläche in Form einer konzentrisch zur Längsachse verlaufenden Zylinderfläche ausgebildet ist und dass die zweite Führungsfläche in Form eine konzentrisch zur Längsachse verlaufenden Hohlzylinderfläche ausgebildet ist. So kann insbesondere eine optimale Führung parallel zur Längsachse erreicht werden, unabhängig von einer Relativposition des Spanwerkzeugs und der Konusschutzeinrichtung voneinander. Die Zylinderfläche sowie die Hohlzylinderfläche können idealerweise so bemessen sein, dass sie nur ein geringes Spiel aufweisen, sodass eine optimale Führung des Spanwerkzeugs durch die Konusschutzeinrichtung möglich ist.

Um eine Montage des medizinischen Instrumentariums zu vereinfachen, insbesondere ein in Eingriff Bringen des Spanwerkzeugs mit der Konusschutzeinrichtung im Bereich der Konusschutzhülse, ist es günstig, wenn sich an die erste Führungsfläche proximalseitig eine sich in proximaler Richtung verjüngende Außenkonusfläche anschließt. Diese Außenkonusfläche kann insbesondere zum Führen der Hohlzylinderfläche des Spanwerkzeugs zur Zylinderfläche der Konusschutzeinrichtung hin unterstützen.

Vorzugsweise ist der Tiefenanschlag proximalseitig der ersten Führungsfläche ausgebildet, insbesondere proximalseitig der Außenkonusfläche. So kann insbesondere eine Beschädigung der ersten Führungsfläche durch das Spanwerkzeug sicher verhindert werden.

Damit eine Bearbeitungstiefe des Spanwerkzeugs relativ zur Konusschutzeinrichtung für einen Operateur auf einfache Weise erkennbar ist, ist es günstig, wenn an der Konusschutzeinrichtung mindestens eine Tiefenmarkierung angeordnet oder ausgebildet ist zum Angeben eines Abstands von einem Gelenkzentrum der Gelenkendoprothese. Insbesondere können hier auch zwei, drei, vier oder mehr Tiefenmarkierungen vorgesehen sein, um einen Operateur bei einer Implantation zu unterstützen.

Günstig ist es, wenn die mindestens eine Tiefenmarkierung in Form einer ringförmigen Markierung ausgebildet ist. Diese kann insbesondere die Längsachse umgebend ausgebildet sein. Beispielsweise kann sie in Form einer Ringnut, eines Ringvorsprungs oder einer durch Laserbeschriftung aufgebrachten Markierung ausgebildet sein. Derartige Tiefenmarkierungen sind für einen Operateur insbesondere gut erkennbar und ermöglichen eine gute Reinigbarkeit des medizinischen Instrumentariums.

Damit die Tiefenmarkierung für einen Operateur auch dann erkennbar ist, wenn das Spanwerkzeug über die Konusschutzhülse in seine distalste Stellung gebracht ist, ist es vorteilhaft, wenn die mindestens eine Tiefenmarkierung proximalseitig des Tiefenanschlags angeordnet oder ausgebildet ist.

Gemäß einer weiteren bevorzugten Ausführungsform umfasst die Konusschutzeinrichtung einen Schaft und ist die Konusschutzhülse distalseitig am Schaft angeordnet oder ausgebildet. Eine solche Ausgestaltung ermöglicht insbesondere eine optimale Handhabung der Konusschutzeinrichtung durch einen Operateur am Schaft, beispielsweise an einem proximalen Ende desselben.

Vorzugsweise ist an einem proximalen Ende der Konusschutzeinrichtung, insbesondere an einem proximalen Ende des Schafts, ein Handhabungsbereich angeordnet oder ausgebildet. So kann ein Operateur die Konusschutzeinrichtung insbesondere in definierter Weise handhaben. Zudem zeigt ihm der Handhabungsbereich auch eine Orientierung des Schafts und damit der Konusschutzeinrichtung an. Dies ist insbesondere vorteilhaft, wenn das medizinische Instrumentarium eingesetzt wird, um eine Gelenkendoprothese minimalinvasiv zu implantieren.

Günstig ist es, wenn der Handhabungsbereich an einem Handhabungselement angeordnet oder ausgebildet ist und wenn das Handhabungselement mit dem proximalen Ende der Konusschutzeinrichtung, insbesondere mit dem proximalen Ende des Schafts, kraft- und/oder form- und/oder stoffschlüssig verbunden ist, insbesondere drehfest. Diese Ausgestaltung ermöglicht es insbesondere, dass ein Operateur die Konusschutzeinrichtung am Handhabungselement erfasst und somit die Konusschutzeinrichtung beispielsweise mit der ersten Implantatkomponente so in Eingriff bringen kann, dass die Konusschutzhülse den Verbindungskonus in gewünschter Weise schützend umgibt.

Vorteilhafterweise ist das Handhabungselement mit dem Schaft unlösbar verbunden. So kann insbesondere vermieden werden, dass sich das Handhabungselement in unerwünschter Weise während eines operativen Eingriffs lösen und verloren gehen kann, beispielsweise im Operationssitus. Zudem kann das Handhabungselement auf diese Weise auch genutzt werden, um das Spanwerkzeug verliersicher mit der Konusschutzeinrichtung zu koppeln. Beispielsweise kann das Handhabungselement einen Anschlag zum Begrenzen einer Bewegung des Spanwerkzeugs relativ zur Konusschutzeinrichtung bilden.

Vorzugsweise bildet das Handhabungselement den proximalen Anschlag. Dieser ermöglicht insbesondere einen kompakten und einfachen Aufbau des medizinischen Instrumentariums.

Günstigerweise definiert eine distale Endfläche des Handhabungselements die zweite Anschlagfläche. So kann die zweite Anschlagfläche eine Bewegung des Spanwerkzeugs in proximaler Richtung begrenzen, beispielsweise im Zusammenwirken mit einer korrespondierenden Anschlagfläche am Spanwerkzeug.

Um die Handhabung des medizinischen Instrumentariums weiter zu verbessern, ist es vorteilhaft, wenn der Handhabungsbereich eine von der Längsachse weg weisende strukturierte Außenfläche aufweist.

Auf einfache Weise lässt sich die Handhabung des Instrumentariums weiter verbessern, wenn die strukturierte Außenfläche parallel zur Längsachse geriffelt ausgebildet ist. So kann ein Operateur die Konusschutzeinrichtung sicher handhaben, ohne vom Handhabungsbereich abzurutschen, auch dann nicht, wenn er Handschuhe trägt.

Günstigerweise ist am Handhabungsbereich ein in proximaler Richtung weisendes Werkzeugkupplungsglied angeordnet oder ausgebildet. Dies ermöglicht es insbesondere, die Konusschutzeinrichtung mit einem Werkzeug zu koppeln, beispielsweise einem Einschraubwerkzeug, um die Konusschutzeinrichtung auf die erste Implantatkomponente aufzuschrauben.

Auf einfache Weise lässt sich das Werkzeugkupplungsglied in Form einer Werkzeugausnehmung oder in Form eines Werkzeugvorsprungs ausbilden. So kann es insbesondere schnell und einfach mit korrespondierenden Werkzeugen in Eingriff gebracht werden, beispielsweise mit zum Einschrauben geeigneten Schraubendreher oder dergleichen.

In einem Operationssaal verfügbare Werkzeuge können insbesondere dann eingesetzt werden, wenn das Werkzeugkupplungsglied in Form eines Mehrkant, eines Vielrund oder eines Schlitzes ausgebildet ist. Beispielsweise können so Werkzeuge in Form von Inbusschlüsseln oder Torx^{®}-Schraubendreher eingesetzt werden, um die Konusschutzeinrichtung auf die erste Implantatkomponente aufzuschrauben.

Um eine optimale Reinigung des Instrumentariums nach einem chirurgischen Eingriff zu ermöglichen, ist es vorteilhaft, wenn die Konusschutzhülse mindestens eine Konusschutzhülsendurchbrechung aufweist. Beispielsweise kann diese in einer Wand der Konusschutzhülse ausgebildet sein und eine langlochartige Form aufweisen. Zum Beispiel kann sich die Durchbrechung parallel zur Längsachse erstrecken.

Vorzugsweise ist das Spanwerkzeug hülsenförmig ausgebildet. Dies ermöglicht es insbesondere, die Konusschutzeinrichtung in das Spanwerkzeug einzuführen beziehungsweise das Spanwerkzeug auf der Konusschutzeinrichtung zu führen.

Ferner kann es günstig sein, wenn das Spanwerkzeug mindestens eine Spanwerkzeugdurchbrechung aufweist. Durch die Spanwerkzeugdurchbrechung kann auch eine Reinigung des Spanwerkzeugs verbessert werden, und zwar insbesondere dann, wenn das Spanwerkzeug unverlierbar an der Konusschutzeinrichtung gesichert gehalten ist.

Gemäß einer weiteren bevorzugten Ausführungsform kann vorgesehen sein, dass sich ein distales Ende der Konusschutzhülse in der Verbindungsstellung distalseitig des Verbindungskonus an der ersten Implantatkomponente abstützt. So kann insbesondere eine Verbindung zwischen der Konusschutzeinrichtung und der ersten Implantatkomponente derart erreicht werden, dass die Konusschutzeinrichtung proximalseitig und distalseitig des Verbindungskonus mit der ersten Implantatkomponente in Kontakt steht, nicht jedoch im Bereich des Verbindungskonus. So kann der Verbindungskonus optimal durch die Konusschutzeinrichtung in der Verbindungsstellung geschützt werden.

Um insbesondere eine Handhabung bei minimalinvasiven Eingriffen zu verbessern, ist es vorteilhaft, wenn das Instrumentarium eine Spanwerkzeugverlängerung zum kraft- und/oder formschlüssigen Koppeln mit einem proximalen Endabschnitt des Spanwerkzeugs umfasst.

Günstig ist es, wenn das Instrumentarium eine Verlängerungkopplungseinrichtung umfasst mit ersten und zweiten Verlängerungkopplungsgliedern, welche einerseits am Spanwerkzeug und andererseits an der Spanwerkzeugverlängerung angeordnet oder ausgebildet sind, und wenn die ersten und zweiten Verlängerungkopplungsglieder in einer Verlängerungkopplungsstellung kraft- und/oder formschlüssig in Eingriff stehen und in einer Trennstellung außer Eingriff stehen. Eine solche Verlängerungkopplungseinrichtung ermöglicht eine einfache und sichere Kopplung der Spanwerkzeugverlängerung mit dem Spanwerkzeug. Insbesondere kann die Spanwerkzeugverlängerung derart ausgebildet sein, dass sie unabhängig von einer Relativstellung des Spanwerkzeugs und der Konusschutzeinrichtung zueinander über ein proximales Ende der Konusschutzeinrichtung vorsteht. Dies ermöglicht es insbesondere, die Konusschutzeinrichtung mit der ersten Implantatkomponente zu verbinden, und dann die Spanwerkzeugverlängerung beispielsweise über ein proximales Ende der Konusschutzeinrichtung an ein proximales Ende des Spanwerkzeugs heranzuführen und mit diesem zu koppeln. Ist die Konusschutzeinrichtung in definierter Weise mit der ersten Implantatkomponente verbunden, muss diese von einem Operateur nicht mehr gehalten werden. So ist ein direkter Zugriff auf ein proximales Ende der Konusschutzeinrichtung in der Verbindungsstellung nicht erforderlich.

Auf einfache Weise lassen sich das Spanwerkzeug und die Spanwerkzeugverlängerung miteinander koppeln, wenn die Verlängerungkopplungseinrichtung in Form einer Bajonett- oder Schraubverbindungseinrichtung ausgebildet ist.

Vorteilhafterweise sind die ersten und zweiten Verlängerungkopplungsglieder in Form von in radialer Richtung bezogen auf die Längsachse ausgebildeten Vorsprüngen und korrespondierenden Ausnehmungen ausgebildet. Beispielsweise können vom Spanwerkzeug in radialer Richtung von der Längsachse weg weisend Kopplungszapfen abstehen, welche in entsprechende Bajonettaufnahmen an der Spanwerkzeugverlängerung in der Verlängerungkopplungsstellung eingreifen.

Um die Spanwerkzeugverlängerung über die Konusschutzeinrichtung führen zu können, ist es günstig, wenn die Spanwerkzeugverlängerung einen Hülsenabschnitt umfasst, welcher sich ausgehend von einem distalen Ende der Spanwerkzeugverlängerung in proximaler Richtung erstrecht. So kann ein proximales Ende der Konusschutzeinrichtung, insbesondere deren Schaft, in den Hülsenabschnitt hineingeführt werden.

Vorzugsweise ist der Hülsenabschnitt zum Aufnehmen des Schafts der Konusschutzeinrichtung ausgebildet. Dieser ist dann durch die Spanwerkzeugverlängerung geschützt, wenn diese mit dem Spanwerkzeug in der Verlängerungkopplungsstellung gekoppelt ist.

Günstigerweise ist ein Innendurchmesser des Hülsenabschnitts an einen Außendurchmesser des Handhabungsbereichs angepasst ist zum Führen einer Relativbewegung des Hülsenabschnitts und des Schafts der Konusschutzeinrichtung. So kann insbesondere ein Verkanten der Konusschutzeinrichtung und der Spanwerkzeugverlängerung relativ zueinander sicher vermieden werden.

Um das medizinische Instrumentarium optimal reinigen zu können, ist es vorteilhaft, wenn der Hülsenabschnitt mindestens eine Reinigungsdurchbrechung aufweist. Insbesondere können hier auch zwei, drei, vier oder mehr Reinigungsdurchbrechungen vorgesehen sein. Diese können beispielsweise in Form von langlochartigen Durchbrechungen ausgebildet sein, welche sich parallel zur Längsachse in einer Hülsenwand des Hülsenabschnitts erstrecken.

Auf einfache Weise lässt sich ein Knochen im proximalen Bereich der Kavität, in welche die erste Implantatkomponente implantiert werden soll, bearbeiten, wenn das Spanwerkzeug in Form einer Ahle ausgebildet ist, insbesondere in Form einer Reibahle.

Die eingangs gestellte Aufgabe wird ferner bei einem Gelenkimplantationssystem der eingangs beschriebenen Art erfindungsgemäß dadurch gelöst, dass das Gelenkimplantationssystem eine der oben beschriebenen bevorzugten Ausführungsformen eines medizinischen Instrumentariums umfasst.

Ein Gelenkimplantationssystem der eingangs beschriebenen Art auf diese Weise weiterzubilden weist insbesondere die bereits oben beschriebenen Vorteile auf. Eine Implantation einer modularen Gelenkendoprothese wird für einen Operateur dadurch insbesondere signifikant vereinfacht.

Günstig ist es, wenn die erste Implantatkomponente mindestens ein Implantatkomponentenkopplungselement umfasst zum kraft- und/oder formschlüssigen in Eingriff Bringen mit einem Konusschutzeinrichtungkopplungselement der Konusschutzeinrichtung in der Verbindungsstellung. Auf diese Weise können die erste Implantatkomponente und die Konusschutzeinrichtung in der Verbindungsstellung in definierter Weise miteinander verbunden werden. So kann insbesondere ein Verbindungskonus der ersten Implantatkomponente beim Einsatz des Spanwerkzeugs durch die Konusschutzeinrichtung sicher vor Beschädigungen geschützt werden.

Auf einfache Weise kann das mindestens eine Implantatkomponentenkopplungselement in Form eines Innen- oder Außengewindeabschnitts ausgebildet sein. Insbesondere kann es korrespondierend zu einem Außen- oder Innengewindeabschnitt ausgebildet sein, welcher das Konusschutzeinrichtungkopplungselement bildet.

Vorzugsweise ist die erste Implantatkomponente in Form eines in die Knochenkavität implantierbaren Schafts der modularen Gelenkendoprothese ausgebildet. Beispielsweise kann es sich dabei um einen Schaft einer Revisionsprothese handeln, die zum Austausch eines Femurschafts einer Hüftgelenkendoprothese in die Knochenkavität eingesetzt werden soll.

Vorteilhafterweise ist die Gelenkendoprothese in Form eine Hüft-, Knie- oder Schultergelenkendoprothese ausgebildet. Bei diesen Prothesen können insbesondere modulare Gelenkkomponenten, beispielsweise Schäfte, in dafür geeignete Knochenkavitäten von Röhrenknochen eingesetzt werden.

Die nachfolgende Beschreibung bevorzugter Ausführungsformen der Erfindung dient im Zusammenhang mit den Zeichnungen der näheren Erläuterung. Es zeigen:
- Figur 1:: eine schematische Gesamtansicht eines proximalen Femurs mit einem Ausführungsbeispiel einer in eine Knochenkavität desselben eingesetzten ersten Implantatkomponente und einem Ausführungsbeispiel eines an diese gekoppelten medizinischen Instrumentarium zum Präparieren eines proximalen Bereichs der Knochenkavität;
- Figur 2:: eine schematische Längsschnittansicht des mit der ersten Implantatkomponente gekoppelten Ausführungsbeispiels des medizinischen Instrumentariums mit in proximaler Richtung verschobenem Spanwerkzeug;
- Figur 3:: eine schematische Schnittansicht ähnlich Figur 2, jedoch mit maximal weit in distaler Richtung vorgeschobenem Spanwerkzeug;
- Figur 4:: eine vergrößerte Ansicht des Bereichs A in Figur 3;
- Figur 5:: eine schematische, teilweise durchbrochene Ansicht des Ausführungsbeispiels des medizinischen Instrumentariums in einer Stellung ähnlich der in Figur 2 dargestellten Stellung;
- Figur 6:: eine schematische Explosionsdarstellung des Ausführungsbeispiels eines medizinischen Instrumentariums aus Figur 1;

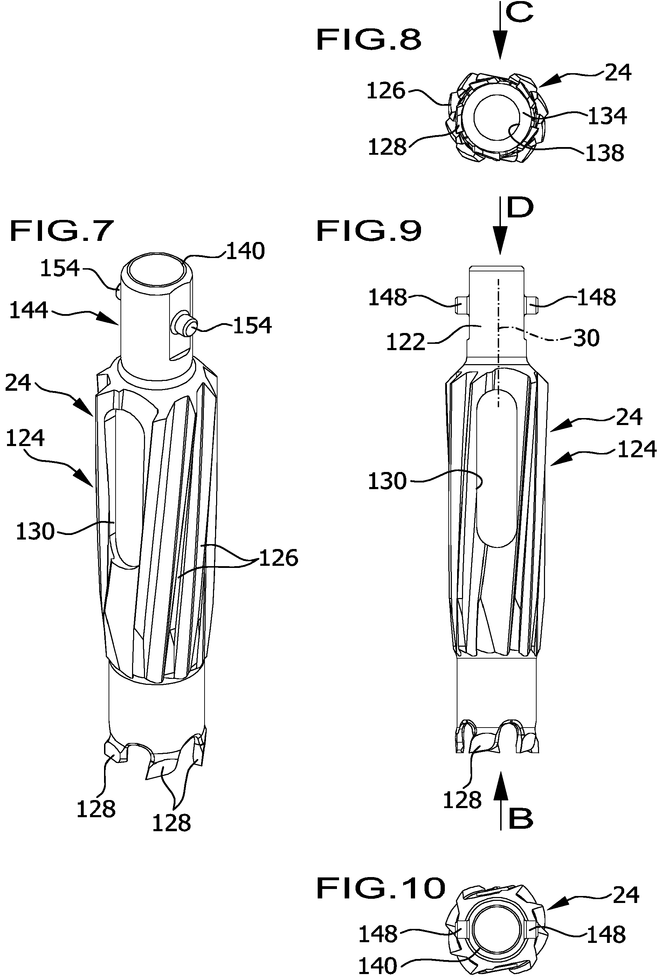

- Figur 7:: eine schematische perspektivische Ansicht eines Ausführungsbeispiels eines Spanwerkzeugs;
- Figur 8:: eine Ansicht des Spanwerkzeugs in Richtung des Pfeils B in Figur 9;
- Figur 9:: eine Ansicht des Spanwerkzeugs in Richtung des Pfeils C in Figur 8;
- Figur 10:: eine Ansicht des Spanwerkzeugs in Richtung des Pfeils D in Figur 9;

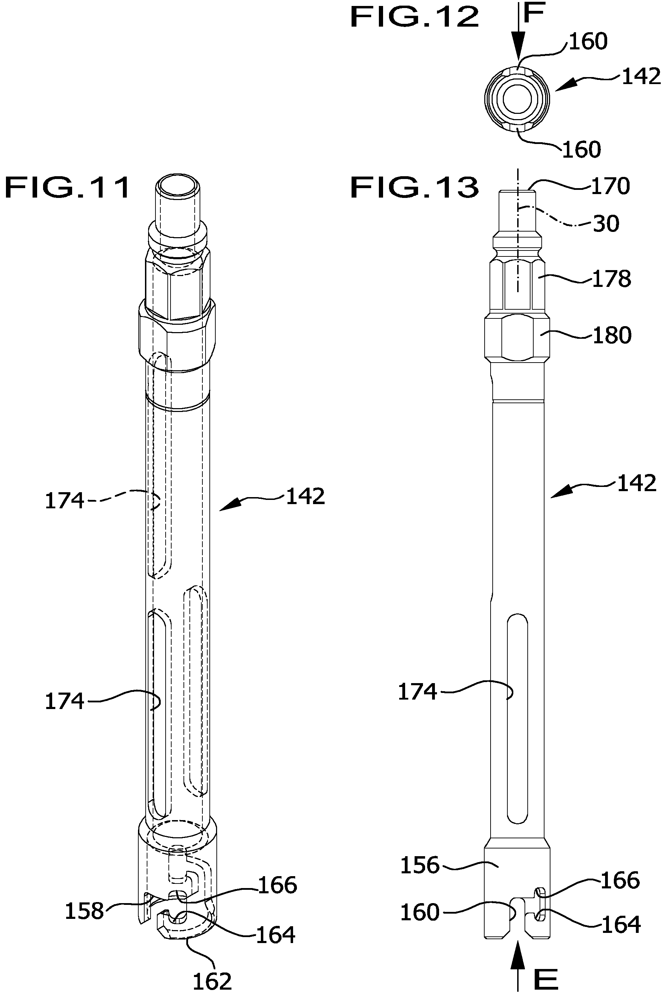

- Figur 11:: eine schematische perspektivische, teilweise durchbrochene Ansicht eines Ausführungsbeispiels einer Spanwerkzeugverlängerung;
- Figur 12:: eine Ansicht der Spanwerkzeugverlängerung aus Figur 13 in Richtung des Pfeils E;
- Figur 13:: eine Ansicht der Spanwerkzeugverlängerung aus Figur 11 in Richtung des Pfeils F in Figur 12; und

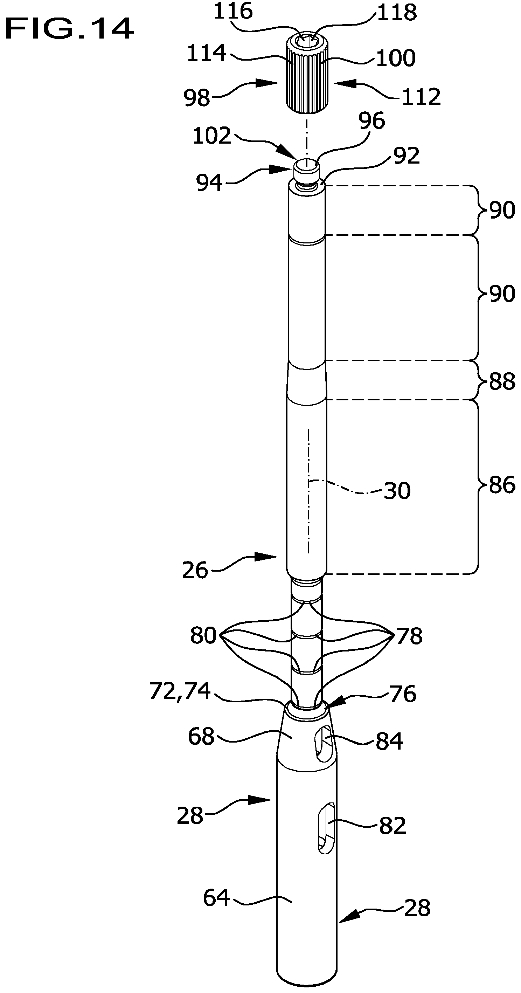

- Figur 14:: eine schematische Explosionsdarstellung des Ausführungsbeispiels der Konusschutzeinrichtung des medizinischen Instrumentariums aus Figur 1.

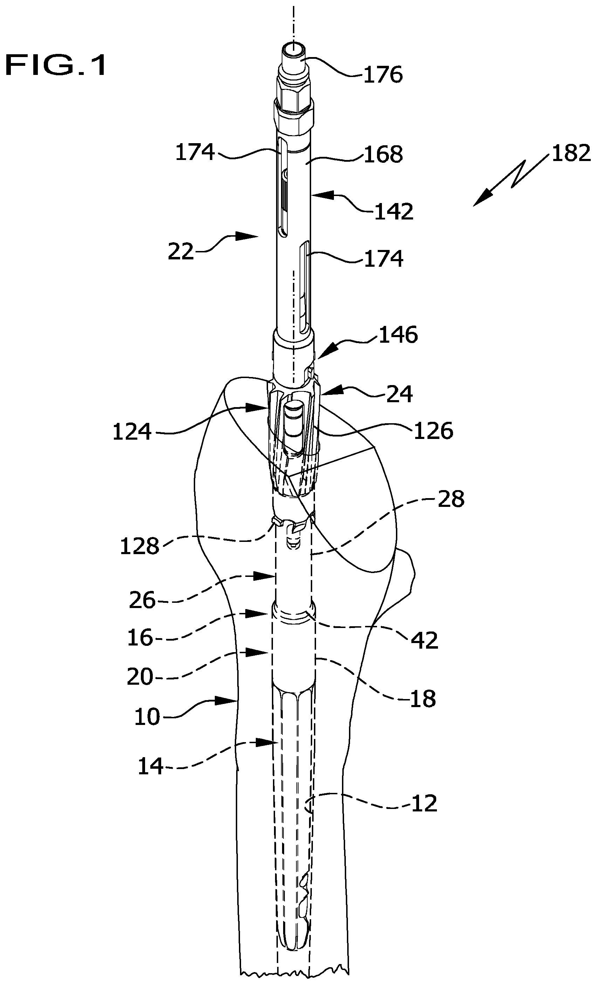

In Figur 1 ist schematisch ein Femur 10 eines Patienten dargestellt, bei welchem der Femurkopf bereits entfernt ist. In eine Kavität 12 des Femurs ist eine erste Implantatkomponente 14 einer Gelenkendoprothese 16 eingesetzt.

Die erste Implantatkomponente 14 ist in Form eines in die Kavität 12 implantierbaren Schafts 18 der modularen Gelenkendoprothese 16 ausgebildet.

Die Gelenkendoprothese 16 ist bei dem in den Figuren dargestellten Ausführungsbeispiel in Form einer Hüftgelenkendoprothese ausgebildet. Bei alternativen Ausführungsbeispielen ist die Gelenkendoprothese 16 in Form von Knie- oder Schultergelenkendoprothesen ausgebildet.

In Figur 1 ist der Schaft 18 mit einem medizinischen Instrumentarium gekoppelt dargestellt, und zwar in einer Verbindungsstellung, die nachfolgend noch näher beschrieben wird.

Das medizinische Instrumentarium 22 umfasst ein Spanwerkzeug 24 und eine Konusschutzeinrichtung 26.

Die Konusschutzeinrichtung 26 umfasst eine Konusschutzhülse 28 und definiert eine Längsachse 30. Die Konusschutzeinrichtung 26 dient dem Zweck, mit der Konusschutzhülse 28 einen von der ersten Implantatkomponente 14 in proximaler Richtung abstehenden Verbindungskonus 32 zu schützen.

In einer Verbindungsstellung, die schematisch in den Figuren 1 bis 4 dargestellt ist, sind die Konusschutzeinrichtung 26 und die erste Implantatkomponente 14 miteinander kraft- und/oder formschlüssig in Eingriff stehend. Die Konusschutzhülse 28 umgibt dabei den Verbindungskonus 32 mindestens teilweise, bei dem in den Figuren dargestellten Ausführungsbeispiel vollständig.

Wie nachfolgend noch eingehend erläutert wird, sind das Spanwerkzeug 24 und die Konusschutzeinrichtung 26 zusammenwirkend ausgebildet zum Führen einer Bewegung des Spanwerkzeugs 24 parallel zur Längsachse 30 und um diese relativ zur Konusschutzeinrichtung 26.

An der ersten Implantatkomponente 14 ist proximalseitig des Verbindungskonus 32 koaxial zur Längsachse ein kurzer zylindrischer Abschnitt 34 ausgebildet, an welchen sich ein kurzer Außengewindeabschnitt 36 anschließt, welcher ein Implantatkomponentenkopplungselement 38 bildet. Proximalseitig schließt sich an den Außengewindeabschnitt 36 ein Werkzeugkupplungselement 40 in Form eines Außenmehrkant an.

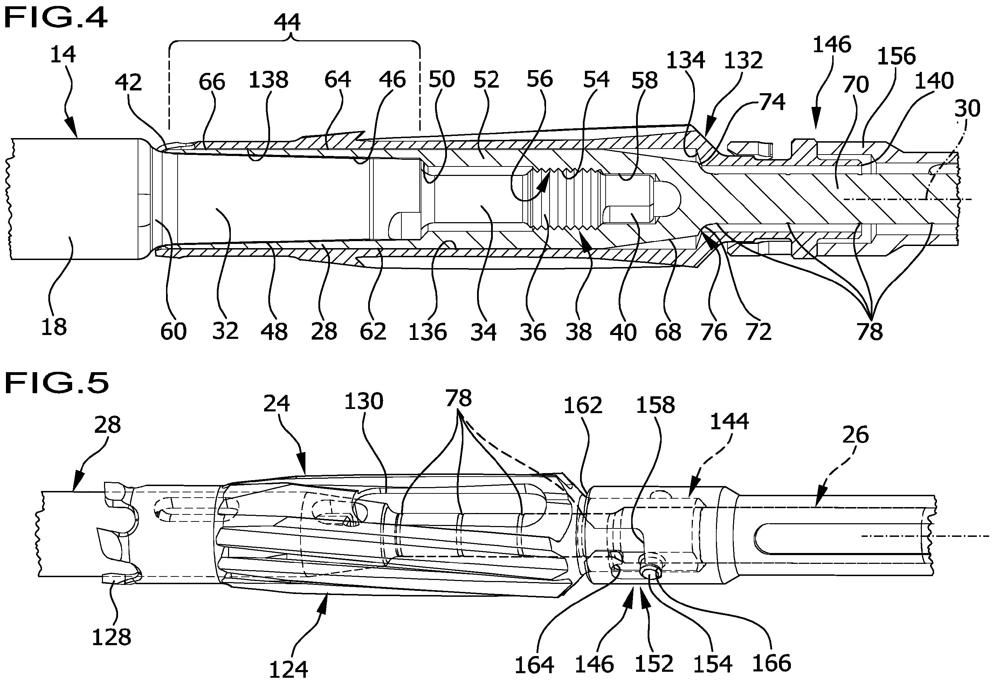

Die Konusschutzhülse 28 definiert ausgehend von ihrem distalen Ende 42 einen Konusschutzabschnitt 44. Der Konusschutzabschnitt 44 definiert eine sich in distaler Richtung erweiternde Konusinnenfläche 46, die wie schematisch in Figur 4 dargestellt in der Verbindungsstellung in Richtung auf eine sich in proximaler Richtung verjüngende Konusaußenfläche 48 des Verbindungskonus 32 hinweist.

Proximalseitig der Konusinnenfläche 46 verjüngt sich ein Innendurchmesser der Konusschutzhülse 28 und bildet eine ringförmige Anschlagschulter 50, an welcher ein Übergangsbereich zwischen dem Verbindungskonus 32 und dem Abschnitt 34 in der Verbindungsstellung anliegt.

An die Anschlagschulter 50 schließt sich proximalseitig ein hohlzylindrischer Abschnitt 52 an. An den Abschnitt 52 schließt sich proximalseitig ein Innengewindeabschnitt 54 an, welcher ein Konusschutzeinrichtungkopplungselement 56 definiert. Der Innengewindeabschnitt 54 ist korrespondierend zum Außengewindeabschnitt 36 ausgebildet.

Proximalseitig des Innengewindeabschnitts 54 ist eine Ausnehmung 58 ausgebildet zum Aufnehmen des Werkzeugkupplungselements 40 in der Verbindungsstellung.

In der beschriebenen Weise umfasst die Konusschutzeinrichtung 26 das Konusschutzeinrichtungkopplungselement 56 zum kraft- und/oder formschlüssigem Koppeln mit dem korrespondierenden Implantatkomponentenkopplungselement 38 der ersten Implantatkomponente 14 in der Verbindungsstellung. Bei dem in den Figuren dargestellten Ausführungsbeispiel ist die Konusschutzhülse 28 derart bemessen, dass sie den Verbindungskonus 32 in der Verbindungsstellung nicht berührt. Mithin ist also zwischen den aufeinander zu weisenden Konusinnen- und Konusaußenflächen 46, 48 in der Verbindungsstellung ein schmaler Spalt ausgebildet.

Das distale Ende 42 der Konusschutzhülse 28 stützt sich in der Verbindungsstellung distalseitig des Verbindungskonus 32 an der ersten Implantatkomponente 14 ab, nämlich an einem Übergangsbereich beziehungsweise an einer zwischen dem Schaft 18 und dem Verbindungskonus 32 ausgebildeten Schulter 60 der ersten Implantatkomponente 14. Dagegen stützt sich bei einer alternativen, in den Figuren nicht dargestellten Ausführungsform das distale Ende 42 der Konusschutzhülse 28 in der Verbindungsstellung distalseitig nicht am Verbindungskonus 32 ab, sondern umgibt die Implantatkomponente 14, insbesondere den Verbindungskonus 32, frei und ohne sie zu berühren, um so eine Beschädigung derselben zu vermeiden.

Ausgehend vom distalen Ende 42 definiert die Konusschutzhülse 28 eine Außenfläche 62, welche eine erste Führungsfläche 64 der Konusschutzeinrichtung 26 zum Führen einer Bewegung des Spanwerkzeugs 24 parallel zur und um die Längsachse 30 relativ zur Konusschutzeinrichtung 26 definiert. Die erste Führungsfläche 64 ist in Form einer konzentrisch zur Längsachse 30 verlaufenden Zylinderfläche 66 ausgebildet.

Die erste Führungsfläche 64 weist eine Länge parallel zur Längsachse 30 auf, die in der Verbindungsstellung bis etwa zu einem proximalseitigen Ende des Außengewindeabschnitts 36 reicht. An die erste Führungsfläche 64 schließt sich proximalseitig eine sich in proximaler Richtung verjüngende Außenkonusfläche 68 an. Die Außenkonusfläche 68 erstreckt sich parallel zur Längsachse 30 in proximaler Richtung etwas über die Ausnehmung 58 hinaus.

Die Konusschutzeinrichtung 26 umfasst ferner einen Schaft 70. Der Schaft 70 weist einen Außendurchmesser auf, welcher kleiner ist als ein Außendurchmesser der Außenkonusfläche 68, sodass im Übergangsbereich zwischen der Außenkonusfläche 68 und dem Schaft 70 eine erste Anschlagfläche 72 in Form einer Ringfläche 74 ausgebildet ist. Die erste Anschlagfläche 72 bildet einen Teil eines Tiefenanschlags 76 der Konusschutzeinrichtung 26 zum Begrenzen einer Bewegung des Spanwerkzeugs 24 parallel zur Längsachse 30 in distaler Richtung.

Ausgehend vom Tiefenanschlag 76 erstreckt sich der Schaft 70 in proximaler Richtung und ist mit drei Tiefenmarkierungen 78 versehen, welche äquidistant zueinander die Längsachse 30 ringförmig umgebend ausgebildet sind. Die Tiefenmarkierungen 78 dienen dem Angeben eines Abstands von einem Gelenkzentrum der Gelenkendoprothese 16.

Bei dem in den Figuren dargestellten Ausführungsbeispiel sind die Tiefenmarkierungen 78 wie erwähnt in Form ringförmiger Markierungen ausgebildet, und zwar in Form jeweils einer Ringnut 80. Bei alternativen Ausführungsbeispielen sind die Tiefenmarkierungen in Form von Ringvorsprüngen oder durch Laserbeschriftung aufgebrachte Markierungen ausgebildet.

Wie beschrieben sind die Tiefenmarkierungen 78 proximalseitig des Tiefenanschlags 76 angeordnet beziehungsweise ausgebildet.

An der Konusschutzhülse 28 sind mehrere Konusschutzhülsendurchbrechungen 82 und 84 ausgebildet. Die Konusschutzhülsendurchbrechung 82 ist im Bereich der ersten Führungsfläche 64 ausgebildet, die Konusschutzhülsendurchbrechung 84 im Bereich der Außenkonusfläche 68. An den Bereich des Schafts 70 mit den Tiefenmarkierungen 78 schließt sich proximalseitig ein Schaftabschnitt 86 an, welcher einen etwas größeren Durchmesser aufweist als der Schaft 70 im Bereich, der mit den Tiefenmarkierungen 78 versehen ist. Der Schaftabschnitt 86 weist eine Länge auf, die in etwa einer Länge des Bereichs des Schafts 70 mit den Tiefenmarkierungen 78 entspricht.

Proximalseitig des Schaftabschnitts 86 verjüngt sich der Schaft 70 auf einem kurzen Übergangsbereich 88 zu einem sich daran proximalseitig anschließenden Schaftabschnitt 90.

Von einer Endfläche 92 des Schaftabschnitts 90 steht koaxial zur Längsachse 30 ein Verbindungsbolzen 94 mit einem im Außendurchmesser verdickten Kopf 96 ab. Der Verbindungsbolzen 94 mit seinem Kopf 96 dient zum Verbinden mit einem Handhabungselement 98, welches in Form einer zylindrischen Hülse 100 ausgebildet ist. Das Handhabungselement 98 ist somit an einem proximalen Ende 102 der Konusschutzeinrichtung 26 angeordnet beziehungsweise ausgebildet. Bei dem in den Figuren dargestellten Ausführungsbeispiel ist das Handhabungselement 98 mit dem proximalen Ende 102 des Schafts 70 kraft- und/oder stoffschlüssig verbunden, und zwar insbesondere drehfest. Hierzu ist ein distales Ende des Handhabungselements 98 umgebördelt und hintergreift den Kopf 96. Mithin ragt also ein Ringflansch des Handhabungselements 98 in eine vom Kopf 96 und der Endfläche 92 begrenzte Ringnut ein.

Das Handhabungselement 98 ist mit dem Schaft 70 unlösbar verbunden. Ein Außendurchmesser des Handhabungselements 98 ist etwas größer als ein Außendurchmesser des Schaftabschnitts 90. Auf diese Weise wird eine in distaler Richtung weisende zweite Ringfläche 104 gebildet, und zwar auf Höhe der Endfläche 92. Die Ringfläche 104 bildet einen Teil einer distalen Endfläche 106 des Handhabungselements 98 und definiert eine zweite Anschlagfläche 108.

Die zweite Anschlagfläche 108 bildet einen Teil eines proximalen Anschlags 110 der Konusschutzeinrichtung 26 zum Begrenzen einer Bewegung des Spanwerkzeugs 24 parallel zur Längsachse 30 in proximaler Richtung. Der proximale Anschlag 110 umfasst die in distaler Richtung weisende zweite Anschlagfläche 108. Auf diese Weise bildet das Handhabungselement 98 den proximalen Anschlag 110.

Die Konusschutzeinrichtung 26 umfasst ferner einen Handhabungsbereich 112, welcher an einem proximalen Ende der Konusschutzeinrichtung 26, nämlich am proximalen Ende 102 des Schafts 70 angeordnet oder ausgebildet ist. Das Handhabungselement 98 umfasst somit den Handhabungsbereich 112.

Für eine optimale Handhabung der Konusschutzeinrichtung 26 ist der Handhabungsbereich 112 mit einer von der Längsachse 30 weg weisenden, strukturierten Außenfläche 114 versehen. Die strukturierte Außenfläche 114 ist parallel zur Längsachse 30 geriffelt ausgebildet.

Am Handhabungsbereich 112, mithin am Handhabungselement 98, ist ein in proximaler Richtung weisendes Werkzeugkupplungsglied 116 angeordnet beziehungsweise ausgebildet. Das Werkzeugkupplungsglied 116 ist bei dem in den Figuren dargestellten Ausführungsbeispiel in Form einer Werkzeugausnehmung 118 ausgebildet. Bei einem alternativen Ausführungsbeispiel ist das Werkzeugkupplungsglied in Form eines Werkzeugvorsprungs ausgebildet, beispielsweise ähnlich dem Werkzeugkupplungselement 40.

Bei dem in den Figuren dargestellten Ausführungsbeispiel ist das Werkzeugkupplungsglied 116 in Form eines Mehrkant 120 ausgebildet, nämlich in Form eines Innenmehrkant. Bei alternativen Ausführungsbeispielen ist das Werkzeugkupplungsglied 116 in Form eines Vielrund oder in Form eines Schlitzes ausgebildet.

Das Spanwerkzeug 24 ist hülsenförmig ausgebildet und umfasst an seinem proximalen Ende einen Kupplungszapfen 122. Es ist zudem in Form einer Reibahle 124 ausgebildet.

Auf einer Außenseite des Spanwerkzeugs 24 sind etwas gegen die Längsachse 30 geneigt Schneiden 126 ausgebildet.

Ein distales Ende des Spanwerkzeugs 24 bilden in distaler Richtung vorstehende Schneidelemente 128. Die Schneidelemente 128 und die Schneiden 126 sind ausgebildet zum spanenden Bearbeiten von Knochen.

Durch den im Bereich des Schaftabschnitts 90 verringerten Durchmesser des Schafts 70 ergibt sich ein größeres Spiel zum Spanwerkzeug 24 im Bereich des Kupplungszapfens 122, so dass das Spanwerkzeug 24 relativ zum Schaft 70 zumindest so weit verkippt werden kann, dass sich das Spanwerkzeug 24 schonend für die den Trochanter des Patienten umgebenden Weichteile in distaler Richtung auf die Konusschutzhülse 28 führen lässt. Die Schneiden 126, auch als Reibahlenzähne bezeichnet, können so bis zum Schaft 70 weggedrückt werden. Dies erleichtert das Vorschieben des Spanwerkzeugs 24 in distaler Richtung, da die Reibahlenzähne nicht so weit seitlich abstehen und sich nicht in den Weichteilen verhaken können.

Zur Verbesserung einer Reinigbarkeit des Spanwerkzeugs 24 weist dieses mindestens eine Spanwerkzeugdurchbrechung 130 auf. Die Spanwerkzeugdurchbrechungen 130, bei dem in den Figuren dargestellten Ausführungsbeispiel sind zwei bezogen auf die Längsachse 30 diametral zueinander angeordnete beziehungsweise ausgebildete Spanwerkzeugdurchbrechungen 130 vorgesehen, erstrecken sich parallel zur Längsachse 30 und sind langlochartig ausgebildet.

Das Spanwerkzeug 24 ist durchgehend hohl ausgebildet. In einem Übergangsbereich 132 zwischen den Schneiden 126 und dem Kupplungszapfen 122 ist im Inneren eine in distaler Richtung weisende Schulter 134 ausgebildet, welche mit dem Tiefenanschlag 76, nämlich der ersten Anschlagfläche 72, zusammenwirkt. Schlägt die Schulter 134 an die erste Anschlagfläche 72 an, ist eine Bewegung des Spanwerkzeugs 24 relativ zur Konusschutzeinrichtung 26 in distaler Richtung unmöglich.

Distalseitig der Schulter 134 ist im Inneren des Spanwerkzeugs 24 eine zweite Führungsfläche 136 ausgebildet. Die zweite Führungsfläche 136 ist korrespondierend zur ersten Führungsfläche 64 der Konusschutzeinrichtung 26 ausgebildet. Mithin ist also die zweite Führungsfläche 136 durch eine auf die Längsachse 30 hin weisende Innenfläche 138 des Spanwerkzeugs 24 ausgebildet.

Die erste Führungsfläche 64 und die zweite Führungsfläche 136 sind so bemessen, dass das Spanwerkzeug 24 parallel zur Längsachse 30 relativ zur Konusschutzhülse 28 verschiebbar und um die Längsachse 30 verdrehbar ist. Die Führungsflächen 64 und 136 gleiten dabei aneinander ab.

Ein Innendurchmesser des Spanwerkzeugs 24 im Bereich des Kupplungszapfens 122 ist an einen Innendurchmesser des Schaftabschnitts 86 angepasst.

Das Spanwerkzeug 24 und die Konusschutzeinrichtung 26 sind bei dem in den Figuren dargestellten Ausführungsbeispiel unverlierbar aneinander gesichert. Wie beschrieben bildet das Handhabungselement 98 mit seiner distalen Endfläche 106 den proximalen Anschlag 110 für das Spanwerkzeug 24. Die zweite Ringfläche 104 wirkt mit einer proximalen Endfläche 140 des Spanwerkzeugs 24 zusammen und begrenzt eine Bewegung desselben in proximaler Richtung, wenn die proximale Endfläche 140 an der zweiten Anschlagfläche 108 anschlägt.

In der beschriebenen Weise bilden das Spanwerkzeug 24 und die Konusschutzeinrichtung 26 eine Einheit, sind jedoch relativ zueinander parallel zur Längsachse 30 axial verschiebbar und um die Längsachse 30 verdrehbar.

Das Instrumentarium 22 umfasst optional eine Spanwerkzeugverlängerung 142 zum kraft- und/oder formschlüssigen Koppeln mit einem proximalen Endabschnitt 144 des Spanwerkzeugs 24. Der proximale Endabschnitt 144 wird definiert durch den Kupplungszapfen 122.

Das Instrumentarium 22 umfasst eine Verlängerungkopplungseinrichtung 146. Die Verlängerungkopplungseinrichtung 146 umfasst erste Verlängerungkopplungsglieder 148 und zu diesen korrespondierende zweite Verlängerungkopplungsglieder 150. Die ersten Verlängerungkopplungsglieder 148 sind am Spanwerkzeug 24 angeordnet beziehungsweise ausgebildet. Die zweiten Verlängerungkopplungsglieder 150 sind an der Spanwerkzeugverlängerung 142 angeordnet beziehungsweise ausgebildet.

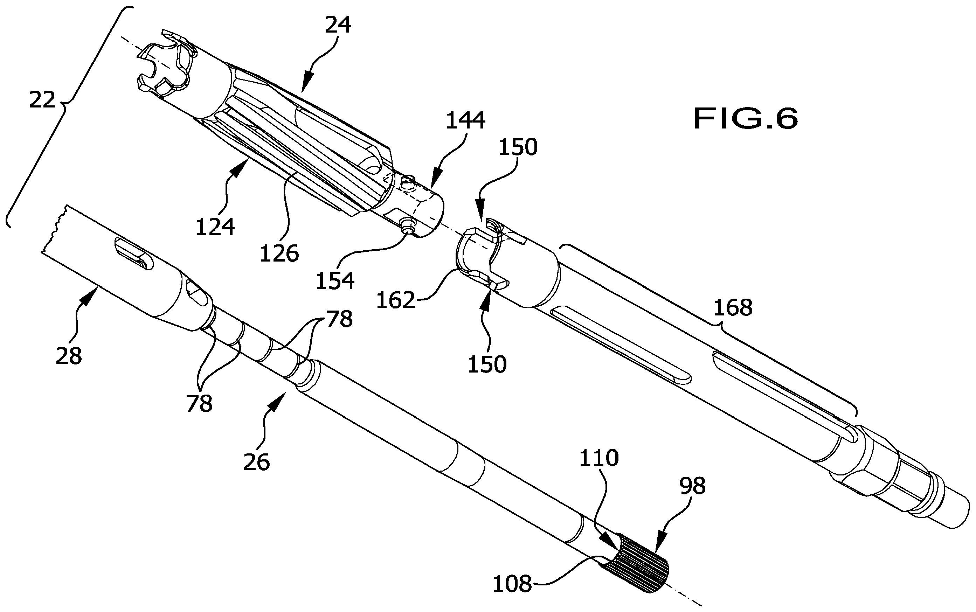

In einer Trennstellung stehen die ersten und zweiten Verlängerungkopplungsglieder 148 und 150 außer Eingriff. Diese Trennstellung ist schematisch in Figur 6 dargestellt.

In einer Verlängerungkopplungsstellung, die schematisch in den Figuren 1 bis 5 dargestellt ist, stehen die ersten und zweiten Verlängerungkopplungsglieder 148 und 150 kraft- und/oder formschlüssig in Eingriff.

Bei dem in den Figuren dargestellten Ausführungsbeispiel ist die Verlängerungkopplungseinrichtung 146 in Form einer Bajonettverbindungseinrichtung 152 ausgebildet. Bei einem in den Figuren nicht dargestellten alternativen Ausführungsbeispiel ist die Verlängerungkopplungseinrichtung 146 in Form einer Schraubverbindungseinrichtung ausgebildet.

Die ersten Verlängerungkopplungsglieder 148 sind in Form von in radialer Richtung von der Längsachse 30 und voneinander weg weisender kurzer zylindrischer Kupplungsvorsprünge 154 ausgebildet. Die zweiten Verlängerungkopplungsglieder 150 sind in einer Hülsenwand 156, welche in der Verlängerungkopplungsstellung den Kupplungszapfen 122 umgibt, in Form L-förmiger Führungsnuten 158, mithin also in Form von Ausnehmungen 160 ausgebildet. Die L-förmigen Führungsnuten 158 erstrecken sich mit einem ersten Schenkel ausgehend von einem distalen Ende 162 parallel zur Längsachse bis zu einem zweiten Schenkel, welcher sich in Umfangsrichtung erstreckt. An einem vom ersten Schenkel weg weisenden freien Ende des zweiten Schenkels sind seitliche, nämlich einerseits in distaler Richtung und anderseits in proximaler Richtung weisend, Vertiefungen 164 und 166 ausgebildet zum Aufnehmen der Kupplungsvorsprünge 154. Die Vertiefungen 164 dienen zum Aufnehmen der Kupplungsvorsprünge 154 beim Zurückziehen der Spanwerkzeugverlängerung 142 relativ zum Spanwerkzeug 24 in proximaler Richtung. Dies ermöglicht es, das Spanwerkzeug 24 unter Zug zu drehen.

Die den Vertiefungen 164 in axialer Richtung gegenüberliegenden Vertiefungen 166 dienen zur Aufnahme der Kupplungsvorsprünge 154 beim Bewegen der Spanwerkzeugverlängerung 42 in distaler Richtung auf das Spanwerkzeug 24 hin. Sie ermöglichen eine drehfeste Kopplung zwischen der Spanwerkzeugverlängerung 142 und dem Spanwerkzeug 24 bei einer Bewegung in distaler Richtung.

An die Hülsenwand 156 schließt sich proximalseitig ein Hülsenabschnitt 168 der Spanwerkzeugverlängerung 142 an. Der Hülsenabschnitt 168 erstreckt sich bis zu einem proximalen Ende 170 der Spanwerkzeugverlängerung 142. Eine Innenfläche 172 des Hülsenabschnitts 168 weist einen Innendurchmesser auf, welcher an einen Außendurchmesser des Handhabungsbereichs 112 angepasst ist zum Führen einer Relativbewegung des Hülsenabschnitts 168 und des Schafts 70 der Konusschutzeinrichtung 26. Das Handhabungselement 98 gleitet an der Innenfläche 172 bei einer Bewegung der Spanwerkzeugverlängerung 142 relativ zur Konusschutzeinrichtung 26 entlang.

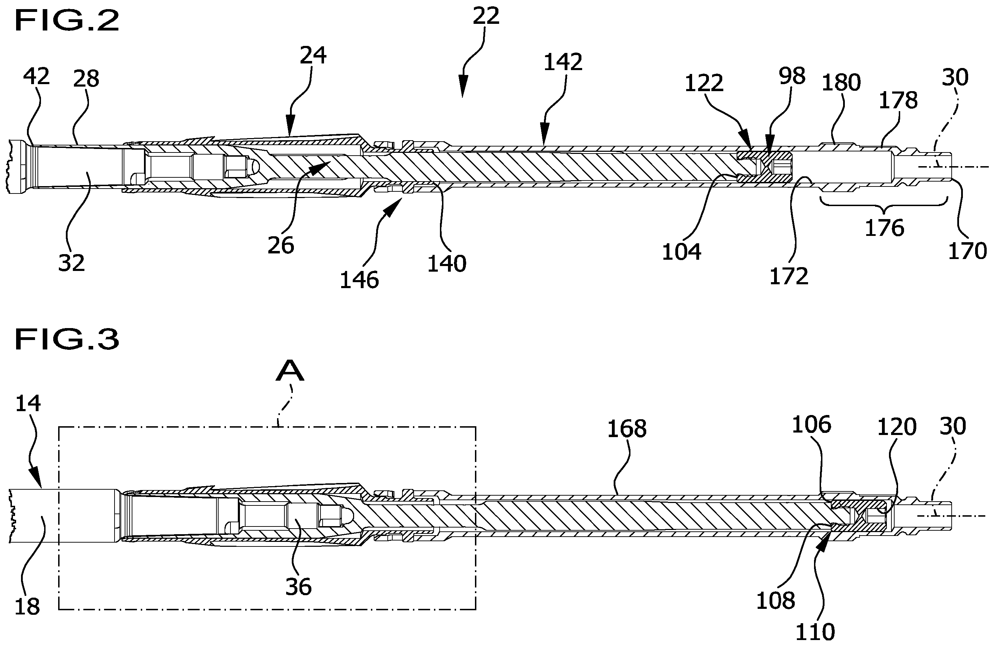

Wie beschrieben und insbesondere in den Figuren 2 und 3 schematisch dargestellt, ist der Hülsenabschnitt 168 zum Aufnehmen des Schafts 70 der Konusschutzeinrichtung 26 ausgebildet.

Am Hülsenabschnitt 168 sind mehrere Reinigungsdurchbrechungen 174 ausgebildet, welche langlochartig geformt sind.

An einem sich ausgehend vom proximalen Ende 170 in distaler Richtung erstreckenden Endabschnitt 176 der Spanwerkzeugverlängerung 172 sind axial zueinander versetzt und aneinander angrenzend zwei Außenmehrkante 178 und 180 ausgebildet, die es ermöglichen, die Spanwerkzeugverlängerung 142 beispielsweise mit einem Maulschlüssel in Eingriff zu bringen und um die Längsachse 30 zu verdrehen.

Die erste Implantatkomponente 14 der modularen Gelenkendoprothese 16 sowie eine zweite, in den Figuren nicht dargestellte Implantatkomponente der modularen Gelenkendoprothese 16 und das medizinische Instrumentarium 22 sind von einem Gelenkimplantationssystem 182 umfasst. Die zweite Implantatkomponente kann beispielsweise in Form einer proximalen Schaftkomponente ausgebildet sein, die, im Falle einer Hüftgelenkendoprothese, mit einem Hals oder direkt einem Gelenkkopf koppelbar ist.

Die Komponenten des Gelenkimplantationssystems 182 sind in der beschriebenen Weise aufeinander abgestimmt, also insbesondere die Konusschutzeinrichtung 26 in Form und Größe auf den Verbindungskonus 32 der ersten Implantatkomponente 14, um den Verbindungskonus 32 in der Verbindungsstellung vor Beschädigungen zu schützen, damit er mit einer zweiten Implantatkomponente final eine selbsthemmende und gegebenenfalls zusätzlich gesicherte Verbindung eingehen kann.

Das Gelenkimplantationssystem 182 ist optional modular ausgebildet und umfasst unterschiedliche erste Implantatkomponenten 14, die sich in Form und/oder Größe voneinander unterscheiden, insbesondere auch in der Ausgestaltung ihrer Verbindungskonen 32. Zur Bearbeitung eines proximalen Bereichs der Kavität 12 sind dann zu den ersten Implantatkomponenten 14 korrespondierende Instrumentarien 22 mit aneinander insbesondere unverlierbar gesicherten Konusschutzeinrichtungen 26 und Spanwerkzeugen 24 vorgesehen.

Mit dem beschriebenen Instrumentarium 22 können modulare Gelenkendoprothesen 16 in der beschriebenen Weise einfach gehandhabt werden. Insbesondere ist eine Implantation der Gelenkendoprothesen 16 über minimalinvasive Zugänge auf einfache und sichere Weise möglich.

### Bezugszeichenliste

- 10: Femur
- 12: Kavität
- 14: erste Implantatkomponente
- 16: Gelenkendoprothese
- 18: Schaft
- 20: Hüftgelenkendoprothese
- 22: medizinisches Instrumentarium
- 24: Spanwerkzeug
- 26: Konusschutzeinrichtung
- 28: Konusschutzhülse
- 30: Längsachse
- 32: Verbindungskonus
- 34: Abschnitt
- 36: Außengewindeabschnitt
- 38: Implantatkomponentenkopplungselement
- 40: Werkzeugkopplungselement
- 42: distales Ende
- 44: Konusschutzabschnitt
- 46: Konusinnenfläche
- 48: Konusaußenfläche
- 50: Anschlagschulter
- 52: Abschnitt
- 54: Innengewindeabschnitt
- 56: Konusschutzeinrichtungkopplungselement
- 58: Ausnehmung
- 60: Schulter
- 62: Außenfläche
- 64: erste Führungsfläche
- 66: Zylinderfläche
- 68: Außenkonusfläche
- 70: Schaft
- 72: erste Anschlagfläche
- 74: Ringfläche
- 76: Tiefenanschlag
- 78: Tiefenmarkierung
- 80: Ringnut
- 82: Konusschutzhülsendurchbrechung
- 84: Konusschutzhülsendurchbrechung
- 86: Schaftabschnitt
- 88: Übergangsbereich
- 90: Schaftabschnitt
- 92: Endfläche
- 94: Verbindungsbolzen
- 96: Kopf
- 98: Handhabungselement
- 100: Hülse
- 102: proximales Ende
- 104: zweite Ringfläche
- 106: distale Endfläche
- 108: zweite Anschlagfläche
- 110: proximaler Anschlag
- 112: Handhabungsbereich
- 114: Außenfläche
- 116: Werkzeugkupplungsglied
- 118: Werkzeugausnehmung
- 120: Mehrkant
- 122: Kupplungszapfen
- 124: Reibahle
- 126: Schneide
- 128: Schneidelemente
- 130: Spanwerkzeugdurchbrechung
- 132: Übergangsbereich
- 134: Schulter
- 136: zweite Führungsfläche
- 138: Innenfläche
- 140: proximale Endfläche
- 142: Spanwerkzeugverlängerung
- 144: proximaler Endabschnitt
- 146: Verlängerungkopplungseinrichtung
- 148: erstes Verlängerungkopplungsglied
- 150: zweites Verlängerungkopplungsglied
- 152: Bajonettverbindungseinrichtung
- 154: Kupplungsvorsprung
- 156: Hülsenwand
- 158: Führungsnut
- 160: Ausnehmung
- 162: distales Ende
- 164: Vertiefung
- 166: Vertiefung
- 168: Hülsenabschnitt
- 170: proximales Ende
- 172: Innenfläche
- 174: Reinigungsdurchbrechung
- 176: Endabschnitt
- 178: Außenmehrkant
- 180: Außenmehrkant
- 182: Gelenkimplantationssystem

## Patentansprüche

1. Medizinisches Instrumentarium (22), umfassend ein Spanwerkzeug (24) und eine Konusschutzeinrichtung (26), wobei die Konusschutzeinrichtung (26) eine eine Längsachse (30) definierende Konusschutzhülse (28) umfasst und ausgebildet ist zum lösbaren Verbinden mit einer einen Verbindungskonus (32) aufweisenden ersten Implantatkomponente (14) einer Gelenkendoprothese (16) derart, dass die Konusschutzhülse (28) in einer Verbindungsstellung, in welcher die Konusschutzeinrichtung (26) und die erste Implantatkomponente (14) miteinander kraft- und/oder formschlüssig in Eingriff stehen, den Verbindungskonus (32) mindestens teilweise, insbesondere vollständig, umgibt und wobei das Spanwerkzeug (24) die Konusschutzeinrichtung (26) und den Verbindungskonus (32) in einer Bearbeitungsstellung umgibt, **dadurch gekennzeichnet, dass** das Spanwerkzeug (24) und die Konusschutzeinrichtung (26) zusammenwirkend ausgebildet sind zum Führen einer Bewegung des Spanwerkzeugs (24) parallel zur und um die Längsachse (30) relativ zur Konusschutzeinrichtung (26).

2. Medizinisches Instrumentarium nach Anspruch 1, **dadurch gekennzeichnet, dass** die Konusschutzeinrichtung (26) einen Tiefenanschlag (76) umfasst zum Begrenzen einer Bewegung des Spanwerkzeugs (24) parallel zur Längsachse (30) in distaler Richtung,
wobei insbesondere der Tiefenanschlag (76) eine in proximaler Richtung weisende erste Anschlagfläche (72) umfasst, insbesondere in Form einer ersten Ringfläche (74).

3. Medizinisches Instrumentarium nach einem der voranstehenden Ansprüche, **dadurch gekennzeichnet, dass** das Spanwerkzeug (24) und die Konusschutzeinrichtung (26) unverlierbar aneinander gesichert sind.

4. Medizinisches Instrumentarium nach einem der voranstehenden Ansprüche, **dadurch gekennzeichnet, dass** die Konusschutzeinrichtung (26) einen proximalen Anschlag (110) zum Begrenzen einer Bewegung des Spanwerkzeugs (24) parallel zur Längsachse (30) in proximaler Richtung umfasst,
wobei insbesondere der proximale Anschlag (110) eine in distaler Richtung weisende zweite Anschlagfläche (108) umfasst, insbesondere in Form einer zweiten Ringfläche (104).

5. Medizinisches Instrumentarium nach einem der voranstehenden Ansprüche, **dadurch gekennzeichnet, dass** die Konusschutzhülse (28) derart bemessen ist, dass sie den Verbindungskonus (32) in der Verbindungsstellung nicht berührt.

6. Medizinisches Instrumentarium nach einem der voranstehenden Ansprüche, **dadurch gekennzeichnet, dass** die Konusschutzeinrichtung (26) ein Konusschutzeinrichtungkopplungselement (56) umfasst zum kraft- und/oder formschlüssigen Koppeln mit einem korrespondierenden Implantatkomponentenkopplungselement (38) der ersten Implantatkomponente (14) der Gelenkendoprothese (16) in der Verbindungsstellung, wobei insbesondere das Konusschutzeinrichtungkopplungselement (56) in Form eines Innen- (54) oder Außengewindeabschnitts ausgebildet ist.

7. Medizinisches Instrumentarium nach einem der voranstehenden Ansprüche, **dadurch gekennzeichnet, dass** die Konusschutzeinrichtung (26) eine erste Führungsfläche (64) zum Führen einer Bewegung des Spanwerkzeugs (24) parallel zur und um die Längsachse (30) relativ zur Konusschutzeinrichtung (26) umfasst und dass die erste Führungsfläche durch eine Außenfläche (62) der Konusschutzeinrichtung (26) gebildet ist, wobei insbesondere
a) das Spanwerkzeug (24) eine zweite Führungsfläche (136) umfasst, wobei die zweite Führungsfläche (136) korrespondierend zur ersten Führungsfläche (64) ausgebildet ist und wobei die zweite Führungsfläche (136) durch eine auf die Längsachse (30) hin weisende Innenfläche (138) des Spanwerkzeugs (24) ausgebildet ist,
wobei insbesondere die erste Führungsfläche (64) in Form einer konzentrisch zur Längsachse (30) verlaufenden Zylinderfläche (66) ausgebildet ist und dass die zweite Führungsfläche (136) in Form einer konzentrisch zur Längsachse (30) verlaufenden Hohlzylinderfläche ausgebildet ist,
und/oder
b) sich an die erste Führungsfläche (64) proximalseitig eine sich in proximaler Richtung verjüngende Außenkonusfläche (68) anschließt
und/oder
c) der Tiefenanschlag (76) proximalseitig der ersten Führungsfläche (64) ausgebildet ist, insbesondere proximalseitig der Außenkonusfläche (68).

8. Medizinisches Instrumentarium nach einem der voranstehenden Ansprüche, **dadurch gekennzeichnet, dass** die Konusschutzeinrichtung (26) einen Schaft (70) umfasst und dass die Konusschutzhülse (28) distalseitig am Schaft (70) angeordnet oder ausgebildet ist.

9. Medizinisches Instrumentarium nach einem der voranstehenden Ansprüche, **dadurch gekennzeichnet, dass** an einem proximalen Ende der Konusschutzeinrichtung (26), insbesondere an einem proximalen Ende (102) des Schafts (70), ein Handhabungsbereich (112) angeordnet oder ausgebildet ist,
wobei insbesondere der Handhabungsbereich (112)
a) an einem Handhabungselement (98) angeordnet oder ausgebildet ist und wobei das Handhabungselement (98) mit dem proximalen Ende der Konusschutzeinrichtung (26), insbesondere mit dem proximalen Ende (102) des Schafts (70), kraft- und/oder form- und/oder stoffschlüssig verbunden ist, insbesondere drehfest, wobei insbesondere das Handhabungselement (98)
- mit dem Schaft (70) unlösbar verbunden ist
und/oder
- den proximalen Anschlag (110) bildet,
wobei insbesondere eine distale Endfläche (106) des Handhabungselements (98) die zweite Anschlagfläche (108) definiert,
und/oder
b) eine von der Längsachse (30) weg weisende strukturierte Außenfläche (114) aufweist,
wobei insbesondere die strukturierte Außenfläche (114) parallel zur Längsachse (30) geriffelt ausgebildet ist,
und/oder
c) ein in proximaler Richtung weisend angeordnetes oder ausgebildetes Werkzeugkupplungsglied (116) umfasst,
wobei insbesondere das Werkzeugkupplungsglied (116)
- in Form einer Werkzeugausnehmung (118) oder in Form eines Werkzeugvorsprungs ausgebildet ist
und/oder
- in Form eines Mehrkant (120), eines Vielrund oder eines Schlitzes ausgebildet ist.

10. Medizinisches Instrumentarium nach einem der voranstehenden Ansprüche, **dadurch gekennzeichnet, dass** das Spanwerkzeug (24)
a) hülsenförmig ausgebildet ist
und/oder
b) mindestens eine Spanwerkzeugdurchbrechung (130) aufweist.

11. Medizinisches Instrumentarium nach einem der voranstehenden Ansprüche, **dadurch gekennzeichnet, dass** sich ein distales Ende (42) der Konusschutzhülse (28) in der Verbindungsstellung distalseitig des Verbindungskonus (32) an der ersten Implantatkomponente (14) abstützt.

12. Medizinisches Instrumentarium nach einem der voranstehenden Ansprüche, **dadurch gekennzeichnet, dass** das Instrumentarium (22) eine Spanwerkzeugverlängerung (142) umfasst zum kraft- und/oder formschlüssigen Koppeln mit einem proximalen Endabschnitt (144) des Spanwerkzeugs (24).

13. Medizinisches Instrumentarium nach Anspruch 12, **dadurch gekennzeichnet, dass**
a) das Instrumentarium (22) eine Verlängerungkopplungseinrichtung (146) umfasst mit ersten und zweiten Verlängerungkopplungsgliedern (148, 150), welche einerseits am Spanwerkzeug (24) und andererseits an der Spanwerkzeugverlängerung (142) angeordnet oder ausgebildet sind, und dass die ersten und zweiten Verlängerungkopplungsgliedern (148, 150) in einer Verlängerungkopplungsstellung kraft- und/oder formschlüssig in Eingriff stehen und in einer Trennstellung außer Eingriff stehen,
wobei insbesondere
- die Verlängerungkopplungseinrichtung (146) in Form einer Bajonett- (152) oder Schraubverbindungseinrichtung ausgebildet ist
und/oder
- die ersten und zweiten Verlängerungkopplungsglieder (148, 150) in Form von in radialer Richtung bezogen auf die Längsachse ausgebildeten Vorsprüngen (154) und korrespondierenden Ausnehmungen (160) ausgebildet sind,
und/oder
b) die Spanwerkzeugverlängerung (142) einen Hülsenabschnitt (168) umfasst, welcher sich ausgehend von einem distalen Ende (162) der Spanwerkzeugverlängerung (142) in proximaler Richtung erstreckt,
wobei insbesondere
- der Hülsenabschnitt (168) ausgebildet ist zum Aufnehmen des Schafts (70) der Konusschutzeinrichtung (26)
und/oder
- ein Innendurchmesser des Hülsenabschnitts (168) an einen Außendurchmesser des Handhabungsbereichs (112) angepasst ist zum Führen einer Relativbewegung des Hülsenabschnitts (168) und des Schafts (70) der Konusschutzeinrichtung (26)
und/oder
- der Hülsenabschnitt (168) mindestens eine Reinigungsdurchbrechung (174) aufweist.

14. Gelenkimplantationssystem (182) umfassend eine in eine Knochenkavität implantierbare erste Implantatkomponente (14) einer modularen Gelenkendoprothese (16), wobei die erste Implantatkomponente (14) einen sich in proximaler Richtung verjüngenden Verbindungskonus (32) umfasst zum klemmenden Verbinden mit einer zweiten Implantatkomponente der modularen Gelenkendoprothese (16), **dadurch gekennzeichnet, dass** das Gelenkimplantationssystem (182) ein medizinisches Instrumentarium (22) nach einem der voranstehenden Ansprüche umfasst.

15. Gelenkimplantationssystem nach Anspruch 14, **dadurch gekennzeichnet, dass** die erste Implantatkomponente (14)
a) mindestens ein Implantatkomponentenkopplungselement (38) umfasst zum kraft- und/oder formschlüssigen in Eingriff Bringen mit einem Konusschutzeinrichtungkopplungselement (56) der Konusschutzeinrichtung (26) in der Verbindungsstellung,
wobei insbesondere das mindestens eine Implantatkomponentenkopplungselement (38) in Form eines Innen- oder Außengewindeabschnitts (36) ausgebildet ist,
und/oder
b) in Form eines in die Knochenkavität (12) implantierbaren Schafts (18) der modularen Gelenkendoprothese (16) ausgebildet ist
und/oder
c) die Gelenkendoprothese (16) in Form einer Hüft- (20), Knie- oder Schultergelenkendoprothese ausgebildet ist.

## Claims

1. Medical instrument set (22), comprising a cutting tool (24) and a conical protection device (26), wherein the conical protection device (26) comprises a conical protection sleeve (28) defining a longitudinal axis (30) and is configured to releasably connect to a first implant component (14) of a joint endoprosthesis (16) comprising a connecting cone (32) in such a way that the conical protection sleeve (28), in a connecting position in which the conical protection device (26) and the first implant component (14) are in force-locking and/or positive-locking engagement with one another, surrounds the connecting cone (32) at least partially, in particular completely, and wherein the cutting tool (24) surrounds the conical protection device (26) and the connecting cone (32) in a working position, **characterized in that** the cutting tool (24) and the conical protection device (26) are configured cooperating with one another to guide a movement of the cutting tool (24) in parallel with and around the longitudinal axis (30) relative to the conical protection device (26).

2. Medical instrument set in accordance with Claim 1, **characterized in that** the conical protection device (26) comprises a depth stop (76) for delimiting a movement of the cutting tool (24) in parallel with the longitudinal axis (30) in the distal direction,
wherein in particular the depth stop (76) comprises a first stop face (72) facing in the proximal direction, in particular in the form of a first annular face (74).

3. Medical instrument set in accordance with any one of the preceding Claims, **characterized in that** the cutting tool (24) and the conical protection device (26) are captively secured to one another.

4. Medical instrument set in accordance with any one of the preceding Claims, **characterized in that** the conical protection device (26) comprises a proximal stop (110) for delimiting a movement of the cutting tool (24) in parallel with the longitudinal axis (30) in the proximal direction, wherein in particular the proximal stop (110) comprises a second stop face (108) facing in the distal direction, in particular in the form of a second annular face (104).

5. Medical instrument set in accordance with any one of the preceding Claims, **characterized in that** the conical protection sleeve (28) is dimensioned in such a way that it does not contact the connecting cone (32) in the connecting position.

6. Medical instrument set in accordance with any one of the preceding Claims, **characterized in that** the conical protection device (26) comprises a conical protection device coupling element (56) for coupling in a force-locking and/or positive-locking manner to a corresponding implant component coupling element (38) of the first implant component (14) of the joint endoprosthesis (16) in the connecting position,
wherein in particular the conical protection device coupling element (56) is configured in the form of an internal (54) or external thread portion.

7. Medical instrument set in accordance with any one of the preceding Claims, **characterized in that** the conical protection device (26) comprises a first guide face (64) for guiding a movement of the cutting tool (24) in parallel with and around the longitudinal axis (30) relative to the conical protection device (26), and **in that** the first guide face is formed by an outer face (62) of the conical protection device (26),
wherein in particular
a) the cutting tool (24) comprises a second guide face (136), wherein the second guide face (136) is configured corresponding to the first guide face (64), and wherein the second guide face (136) is formed by an inner face (138) of the cutting tool (24) facing toward the longitudinal axis (30),
wherein in particular the first guide face (64) is configured in the form of a cylindrical face (66) extending concentrically with the longitudinal axis (30), and **in that** the second guide face (136) is configured in the form of a hollow cylindrical face extending concentrically with the longitudinal axis (30),
and/or
b) the first guide face (64) is adjoined proximally by an outer cone face (68) tapering in the proximal direction
and/or
c) the depth stop (76) is formed on the proximal side of the first guide face (64), in particular on the proximal side of the outer cone face (68).

8. Medical instrument set in accordance with any one of the preceding Claims, **characterized in that** the conical protection device (26) comprises a shaft (70) and **in that** the conical protection sleeve (28) is arranged or formed distally on the shaft (70).

9. Medical instrument set in accordance with any one of the preceding Claims, **characterized in that** a handling region (112) is arranged or formed on a proximal end of the conical protection device (26), in particular on a proximal end (102) of the shaft (70),
wherein in particular the handling region (112)
a) is arranged or formed on a handling element (98), and wherein the handling element (98) is connected in a force-locking and/or positive-locking and/or materially bonded manner, in particular in a rotationally-fixed manner, to the proximal end of the conical protection device (26), in particular to the proximal end (102) of the shaft (70),
wherein in particular the handling element (98)
- is non-releasably connected to the shaft (70)
and/or
- forms the proximal stop (110),
wherein in particular a distal end face (106) of the handling element (98) defines the second stop face (108),
and/or
b) comprises a structured outer face (114) facing away from the longitudinal axis (30),
wherein in particular the structured outer face (114) is configured fluted in parallel with the longitudinal axis (30),
and/or
c) comprises a tool coupling member (116) arranged or formed facing in the proximal direction,
wherein in particular the tool coupling member (116)
- is configured in the form of a tool recess (118) or in the form of a tool projection
and/or
- is configured in the form of a polygon (120), a multi-lobe, or a slot.

10. Medical instrument set in accordance with any one of the preceding Claims, **characterized in that** the cutting tool (24)
a) is of sleeve-shaped configuration
and/or
b) has at least one cutting tool perforation (130).

11. Medical instrument set in accordance with any one of the preceding Claims, **characterized in that** a distal end (42) of the conical protection sleeve (28) in the connecting position is supported on the first implant component (14) on the distal side of the connecting cone (32).

12. Medical instrument set in accordance with any one of the preceding Claims, **characterized in that** the instrument set (22) comprises a cutting tool extension (142) for coupling to a proximal end portion (144) of the cutting tool (24) in a force-locking and/or positive-locking manner.

13. Medical instrument set in accordance with Claim 12, **characterized in that**
a) the instrument set (22) comprises an extension coupling device (146) having first and second extension coupling members (148, 150), which are arranged or formed on the cutting tool (24) on the one hand and on the cutting tool extension (142) on the other hand, and **in that** the first and second extension coupling members (148, 150) are in engagement in a force-locking and/or positive-locking manner in an extension coupling position and are out of engagement in a separating position,
wherein in particular
- the extension coupling device (146) is configured in the form of a bayonet (152) or screw connection
and/or
- the first and second extension coupling members (148, 150) are configured in the form of projections (154) formed in the radial direction relative to the longitudinal axis and in the form of corresponding recesses (160),
and/or
b) the cutting tool extension (142) comprises a sleeve portion (168), which extends in the proximal direction commencing from a distal end (162) of the cutting tool extension (142),
wherein in particular
- the sleeve portion (168) is configured to accommodate the shaft (70) of the conical protection device (26)
and/or
- an inner diameter of the sleeve portion (168) is adapted to an outer diameter of the handling region (112) for guiding a relative movement of the sleeve portion (168) and the shaft (70) of the conical protection device (26)
and/or
- the sleeve portion (168) comprises at least one cleaning perforation (174).

14. Joint implantation system (182) comprising a first implant component (14) of a modular joint endoprosthesis (16) that is implantable into a bone cavity, wherein the first implant component (14) comprises a connecting cone (32) tapering in the proximal direction for clampingly connecting to a second implant component of the modular joint endoprosthesis (16), **characterized in that** the joint implantation system (182) comprises a medical instrument set (22) in accordance with any one of the preceding Claims.

15. Joint implantation system in accordance with Claim 14, **characterized in that** the first implant component (14)
a) comprises at least one implant component coupling element (38) for being brought into force-locking and/or positive-locking engagement with a conical protection device coupling element (56) of the conical protection device (26) in the connecting position, wherein in particular the at least one implant component coupling element (38) is configured in the form of an internal or external thread portion (36),
and/or
b) is configured in the form of a shaft (18) of the modular joint endoprosthesis (16) that is implantable into the bone cavity (12)
and/or
c) the joint endoprosthesis (16) is configured in the form of a hip (20), knee, or shoulder joint endoprosthesis.

## Revendications

1. Instrument médical (22), comprenant un outil de coupe (24) et un dispositif de protection de cône (26), dans lequel le dispositif de protection de cône (26) comprend un manchon de protection de cône (28) qui définit un axe longitudinal (30) et qui est conçu pour être relié de manière amovible à un premier composant d'implant (14) d'une endoprothèse articulaire (16) présentant un cône de connexion (32) de telle sorte que le manchon de protection de cône (28), dans une position de connexion dans laquelle le dispositif de protection de cône (26) et le premier composant d'implant (14) sont en prise l'un avec l'autre par liaison de force et/ou par complémentarité de forme, entoure au moins partiellement, en particulier complètement, le cône de connexion (32), et l'outil de coupe (24) entoure le dispositif de protection de cône (26) et le cône de connexion (32) dans une position d'usinage, **caractérisé en ce que** l'outil de coupe (24) et le dispositif de protection de cône (26) sont conçus de manière à coopérer pour guider un mouvement de l'outil de coupe (24) parallèlement à l'axe longitudinal (30) et autour de celui-ci par rapport au dispositif de protection de cône (26).

2. Instrument médical selon la revendication 1, **caractérisé en ce que** le dispositif de protection de cône (26) comprend une butée de profondeur (76) destinée à limiter un mouvement de l'outil de coupe (24) parallèlement à l'axe longitudinal (30) dans la direction distale,
dans lequel, en particulier, ladite butée de profondeur (76) comprend une première surface de butée (72) orientée dans la direction proximale, en particulier sous la forme d'une première surface annulaire (74).

3. Instrument médical selon l'une des revendications précédentes, **caractérisé en ce que** l'outil de coupe (24) et le dispositif de protection de cône (26) sont fixés l'un à l'autre de manière imperdable.

4. Instrument médical selon l'une des revendications précédentes, **caractérisé en ce que** le dispositif de protection de cône (26) comprend une butée proximale (110) destinée à limiter un mouvement de l'outil de coupe (24) parallèlement à l'axe longitudinal (30) dans la direction proximale,
dans lequel, en particulier, la butée proximale (110) comprend une deuxième surface de butée (108) orientée dans la direction distale, en particulier sous la forme d'une deuxième surface annulaire (104).

5. Instrument médical selon l'une des revendications précédentes, **caractérisé en ce que** le manchon de protection de cône (28) est dimensionné de telle sorte qu'il ne touche pas le cône de connexion (32) dans la position de connexion.

6. Instrument médical selon l'une des revendications précédentes, **caractérisé en ce que** le dispositif de protection de cône (26) comprend un élément d'accouplement du dispositif de protection de cône (56) destiné à être accouplé par liaison de force et/ou par complémentarité de forme à un élément d'accouplement de composant d'implant (38) correspondant du premier composant d'implant (14) de l'endoprothèse articulaire (16) dans la position de connexion,
dans lequel, en particulier, l'élément d'accouplement du dispositif de protection de cône (56) est réalisé sous la forme d'une section de filetage intérieur (54) ou extérieur.

7. Instrument médical selon l'une des revendications précédentes, **caractérisé en ce que** le dispositif de protection de cône (26) comprend une première surface de guidage (64) destinée à guider un mouvement de l'outil de coupe (24) parallèlement à l'axe longitudinal (30) et autour de celui-ci par rapport au dispositif de protection de cône (26), et **en ce que** la première surface de guidage est formée par une surface extérieure (62) du dispositif de protection de cône (26),
dans lequel, en particulier,
a) l'outil de coupe (24) comprend une deuxième surface de guidage (136), laquelle deuxième surface de guidage (136) est réalisée de manière correspondante à la première surface de guidage (64), et la deuxième surface de guidage (136) est formée par une surface intérieure (138) de l'outil de coupe (24) orientée vers l'axe longitudinal (30),
dans lequel, en particulier, la première surface de guidage (64) est réalisée sous la forme d'une surface cylindrique (66) qui s'étend concentriquement à l'axe longitudinal (30), et la deuxième surface de guidage (136) est réalisée sous la forme d'une surface cylindrique creuse qui s'étend concentriquement à l'axe longitudinal (30),
et/ou
b) une surface conique extérieure (68) qui se rétrécit dans la direction proximale est adjacente à la première surface de guidage (64) du côté proximal,
et/ou
c) la butée de profondeur (76) est réalisée du côté proximal de la première surface de guidage (64), en particulier du côté proximal de la surface conique extérieure (68).

8. Instrument médical selon l'une des revendications précédentes, **caractérisé en ce que** le dispositif de protection de cône (26) comprend une tige (70) et **en ce que** le manchon de protection de cône (28) est disposé ou formé du côté distal sur la tige (70).

9. Instrument médical selon l'une des revendications précédentes, **caractérisé en ce qu'**une zone de manipulation (112) est disposée ou formée à une extrémité proximale du dispositif de protection de cône (26), en particulier à une extrémité proximale (102) de la tige (70), dans lequel, en particulier, la zone de manipulation (112)
a) est disposée ou formée sur un élément de manipulation (98), et l'élément de manipulation (98) est relié à l'extrémité proximale du dispositif de protection de cône (26), en particulier à l'extrémité proximale (102) de la tige (70), par liaison de force et/ou par complémentarité de forme et/ou par liaison de matière, en particulier de manière non rotative,
dans lequel, en particulier, l'élément de manipulation (98)
- est relié de manière non amovible à la tige (70)
et/ou
- forme la butée proximale (110),
dans lequel, en particulier, une surface d'extrémité distale (106) de l'élément de manipulation (98) définit la deuxième surface de butée (108),
et/ou
b) présente une surface extérieure structurée (114) orientée à l'opposé de l'axe longitudinal (30),
dans lequel, en particulier, la surface extérieure structurée (114) est cannelée parallèlement à l'axe longitudinal (30),
et/ou
c) comprend un organe d'accouplement d'outil (116) disposé ou formé en étant orienté dans la direction proximale,
dans lequel, en particulier, l'organe d'accouplement d'outil (116)
- est réalisé sous la forme d'un évidement d'outil (118) ou sous la forme d'une saillie d'outil
et/ou
- est réalisé sous la forme d'un polygone (120), d'un profilé multirond ou d'une fente.

10. Instrument médical selon l'une des revendications précédentes,
**caractérisé en ce que** l'outil de coupe (24)
a) est réalisé en forme de manchon
et/ou
b) présente au moins une ouverture traversante de l'outil de coupe (130).

11. Instrument médical selon l'une des revendications précédentes, **caractérisé en ce qu'**une extrémité distale (42) du manchon de protection de cône (28) prend appui, dans la position de connexion, du côté distal du cône de connexion (32) sur le premier composant d'implant (14).

12. Instrument médical selon l'une des revendications précédentes, **caractérisé en ce que** l'instrument (22) comprend une rallonge d'outil de coupe (142) destinée à être accouplée par liaison de force et/ou par complémentarité de forme à une section d'extrémité proximale (144) de l'outil de coupe (24).

13. Instrument médical selon la revendication 12, **caractérisé en ce que**
a) l'instrument (22) comprend un dispositif d'accouplement de rallonge (146) avec des premiers et deuxièmes organes d'accouplement de rallonge (148, 150), qui sont disposés ou formés d'une part sur l'outil de coupe (24) et d'autre part sur la rallonge d'outil de coupe (142), et **en ce que** les premiers et deuxièmes organes d'accouplement de rallonge (148, 150) sont en prise par liaison de force et/ou par complémentarité de forme dans une position d'accouplement de rallonge et sont hors prise dans une position de séparation,
dans lequel, en particulier,
- le dispositif d'accouplement de rallonge (146) est réalisé sous la forme d'un dispositif de liaison à baïonnette (152) ou à vis,
et/ou
- les premiers et deuxièmes organes d'accouplement de rallonge (148, 150) sont réalisés sous la forme de saillies (154) formées dans la direction radiale par rapport à l'axe longitudinal et d'évidements correspondants (160),
et/ou
b) la rallonge d'outil de coupe (142) comprend une section de manchon (168), qui s'étend dans la direction proximale à partir d'une extrémité distale (162) de la rallonge d'outil de coupe (142),
dans lequel, en particulier,
- la section de manchon (168) est conçue pour recevoir la tige (70) du dispositif de protection de cône (26)
et/ou
- un diamètre intérieur de la section de manchon (168) est adapté à un diamètre extérieur de la zone de manipulation (112) pour guider un mouvement relatif de la section de manchon (168) et de la tige (70) du dispositif de protection de cône (26)
et/ou
- la section de manchon (168) présente au moins une ouverture traversante de nettoyage (174).

14. Système d'implantation articulaire (182) comprenant un premier composant d'implant (14) d'une endoprothèse articulaire modulaire (16) implantable dans une cavité osseuse, le premier composant d'implant (14) comprenant un cône de connexion (32) qui se rétrécit dans la direction proximale pour être relié par serrage à un deuxième composant d'implant de l'endoprothèse articulaire modulaire (16), **caractérisé en ce que** le système d'implantation articulaire (182) comprend un instrument médical (22) selon l'une des revendications précédentes.

15. Système d'implantation articulaire selon la revendication 14, **caractérisé en ce que** le premier composant d'implant (14)
a) comprend au moins un élément d'accouplement de composant d'implant (38) destiné à être mis en prise par liaison de force et/ou par complémentarité de forme avec un élément d'accouplement du dispositif de protection de cône (56) du dispositif de protection de cône (26) dans la position de connexion,
dans lequel, en particulier, ledit au moins un élément d'accouplement de composant d'implant (38) est réalisé sous la forme d'une section de filetage intérieur ou extérieur (36),
et/ou
b) est réalisé sous la forme d'une tige (18) de l'endoprothèse articulaire modulaire (16) implantable dans la cavité osseuse (12),
et/ou
c) l'endoprothèse articulaire (16) est réalisée sous la forme d'une endoprothèse d'articulation de la hanche (20), du genou ou de l'épaule.
